# EUROPEAN PATENT SPECIFICATION

(11) **EP 1 733 759 B1**
(45) Date of publication and mention of the grant of the patent: **05.09.2012**
(21) Application number: 05012202.7
(22) Date of filing: 07.06.2005
(51) Int. Cl.: A61Q 5/02, A61Q 5/06, A61Q 5/12, A61K 8/41, A61K 8/68, A61K 8/97

(54) **Composition for hair comprising a liquid extract from Bambusa vulgaris**
Haarzusammensetzung enthaltend ein flüssigen Bambusa vulgaris Extrakt
Composition capillaire contenant un extrait liquide de Bambusa vulgaris

(43) Date of publication of application: 20.12.2006
(73) Proprietor: Kao Germany GmbH, 64297 Darmstadt (DE)
(72) Inventor: Hermes, Frank, 64342 Seehelm-Jugenheim (DE); Bräutigam, Ina, 64297 Darmstadt (DE); Lehn, Jutta, 64285 Darmstadt (DE); Weichaus, Dirk, 64404 Bickenbach (DE); Cajan, Christine, 56130 Bad Ems (DE); Shiga, Akira, Sakura-shi Tochigi 329-1311 (JP)

(56) References cited:
- EP-A- 0 573 141
- WO-A-99/27900
- DE-A1- 19 941 819
- DE-C1- 10 139 612
- GB-A- 1 066 927
- US-A1- 2004 241 114

## Description

The present invention is related to a hair styling composition for hair comprising extract from Bambusa vulgaris in liquid form, and at least one film forming polymer.

Hair care compositions have been found their application for many decades. Although the state of the art is quite well established there is still need for improvements.

Natural ingredients have always been attractive to the consumer. Compositions containing natural ingredients especially derived from the edible ones have always been regarded as safer than any other composition offered mainly based on synthetic raw materials. Designing modem cosmetic formulations solely on the basis of natural ingredients will probably take more time in the future.

In cosmetic magazines, periodicals as well as textbooks, the use of natural ingredients has been shown together with synthetic raw materials. Chemically modified active ingredients gained from natural resources are as well found their applications in cosmetics.

The plant bamboo has been known for its rich nutritious properties. Although it is a grass plant, it groves to a height of up to 40 m very fast. Parts of Bamboo plants have also been used as a building material for houses. Owing to its extreme flexibility, plastic industry has been working on the use of Bamboo fibres to reinforce material's mechanical properties.

WO 2004/017934 A1 is on skin smoothing compositions based on plant extracts. Ingredients prepared from Bambusa vulgaris mentioned are mainly in powder form. In the document nothing is mentioned on the use of extracts from Bambusa vulgaris in compositions for keratin fibres, especially hair.

EP 573 141 A2, US 2004/0241114 A1, WO 99/27900 A1, DE 101 39 612 C1, DE 199 41 819 A1 and GB 1,066,927 disclose cosmetic compositions comprising Bamboo extracts. In none of the documents a hair styling composition comprising at least one film forming polymer is disclosed in combination with liquid bamboo extract.

It has surprisingly been found out that incorporation of liquid extracts from Bambusa vulgaris into hair styling compositions for hair improves hair conditioning properties and especially hair shine.

Thus, the subject of the present invention is providing hair styling compositions for hair comprising liquid extracts from Bambusa vulgaris, and at least one film forming polymer at a concentration of 0.5 to 25% by weight, calculated to total composition.

It is yet another subject of the present invention that the use of compositions comprising liquid extracts from Bambusa vulgaris and at least one forming polymer for improving hair shine.

Liquid extracts from Bambusa vulgaris are commercially available from the company Cosmetochem. Briefly, the extracts are prepared from the plant Bambusa vulgaris using a solvent comprising C₂-C₄ alcohol and/or ethylene or butylenes glycol derivatives and/or water. Preferred solvents for preparing the extracts are isopropyl alcohol and propylene glycol either as it is or in admixture with water. The extracts comprise typically the solvents at a concentration of at least 50%, preferably 60% and more preferably 70% by weight calculated to total extract. The extracts comprise active matter at a concentration of at least 1%, preferably 2%, more preferably 3%, most preferably 5% by weight calculated to total composition of extract.

Compositions of the present invention comprise liquid extracts from Bambusa vulgaris at a concentration of 0.01 to 10%, preferably 0.01 to 5%, more preferably 0.01 to 3 by weight calculated to total composition and as dry matter of the extract.

Styling compositions for hair according to the present invention are any of those well-known types such as gels, foams, styling sprays either aerosol or non-aerosol, styling waxes. Those compositions used especially after shampooing can be applied to hair either as a leave-in or as a rinse off compositions. Oily substances as conditioners and/or for any other purpose depending on the purpose of the formulation according to the present invention are selected from silicone oils either volatile or non-volatile, natural and synthetic oils. Among silicone oils those can be added to the compositions include dimethicone, dimethiconol, polydimethylsiloxane, cyclomethicone and phenyltrimethicone. Commercially, they are available from various companies for example Dow Coming with the known DC series, Wacker Chemie and Toray silicones. All commercially available non volatile silicones are suitable in the compositions of the present invention. Examples to those are DC 200 series, DC1401, DC 1403, DC 1501 and DC 1503. Concentration of silicone oils in the compositions of the present invention is typically between 0.1 to 20% by weight calculated to total composition. Cationic silicones know with INCI name as amodimethicone can as well be contained in the compositions of the present invention. Commercially it is available under the trade name DC 929 in emulsified form in mixture with a nonionic surfactant and a cationic surfactant.

Natural oils suitable are such as olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof, as well as mineral oils such as paraffin oil and petrolatum.

Lipophilic compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters such as PEG-7-glyceryl cocoate, cetyl palmitate.)

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula,

R₁₂ CO (O CH₂ CH₂)ₙ OH

R₁₂ CO (O CH₂ CH₂)ₙ O OC R₁₃

where R₁₂ and R₁₃ are independent from each other saturated, unsaturated or branched or non-branched alkyl chain with 7 to 21 C atoms and n is typically 2-100.

Typical concentration range for any of the additional conditioners mentioned above other than cationic conditioning compounds can be 0.01 - 15% by weight, preferably 0.05 - 5% by weight calculated to the total composition.

The compositions according to the invention can also comprise further agents, such as protein hydrolyzates and polypeptides, e.g. keratin hydrolyzates, collagen hydrolyzates of the type "Nutrilan^{®}" or elastin hydrolyzates, as well as, in particular vegetable, optionally cationized protein hydrolyzates, for example "Gluadin^{®}".

Additional natural plant extracts can as well form part of the compositions of the present invention. Those are incorporated usually in an amount of 0.01 % to 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn.

Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4^{th} Ed..

Among the natural ingredients in the form of an extract, especially preferred component of the composition according to the invention is green tea extract. This tea extract is obtained from the leaves, leaf buds and tender stems of the tea shrub, Camellia sinensis or Camellia oleifera, by aqueous or hydro-alcoholic extraction and subsequent spray-drying. In difference to black tea, green tea is a non-fermented product obtained from the Thea sinensis or Thea assamica species. An overview of the biological and pharmacological effects of green tea and the ingredients thereof can be found, e.g., in an article by A. Pistorius, "Seifen-Öle-Fette-Wachse-Journal", Volume 122., No. 7/1996, pages 468 to 471, to which reference is made. The content of green tea extract is variable in the compositions according to the invention. It preferably ranges from 0.01 % to 10 %, preferably 0.05 % to 5% by weight, calculated to the total composition and the pulverulent extract.

The compositions may contain organic solvents such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition is very much dependent on the product and application form. For example for a shampoo or an emulsion conditioner the concentration should not exceed 5% by weight. It should be noted that penetration enhancers are useful for both cleansing and after shampoo conditioning preparations. It is obvious that the concentration in the cleansing compositions is usually lower than in the conditioning preparations. However, for a styling composition or again a detangling composition applied with a pump foamer, the concentration may be as high as i.e. 50% by weight, or an aerosol composition may be based on alcohol and/or other suitable organic solvent.

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds:
4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof,
4-methoxycinnamic acid and the esters and salts thereof, salicylic acid and the esters and salts thereof, 2.4₋dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone. 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebomane-2-one and the salts thereof and/or 3-(4'-methyl benzylidene)-DL-campher.

The preferred amount of the UV-absorber ranges from 0.01 % to 2.5%, more preferably from 0.05 % to 1 % by weight, calculated to the total composition.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to the total composition. It should be noted without limiting their use that the solubilizers are especially used in the clear cleansing and/or conditioning preparations in order to overcome the turbidity caused by addition of lipophilic materials.

The compositions of the present invention can comprise hair-restructuring agents. The hair restructuring agents preferred are especially the ones disclosed in the German patent DE 197 51 550 C2.

One of the known hair restructuring agents is ceramide type of compound with the general formula 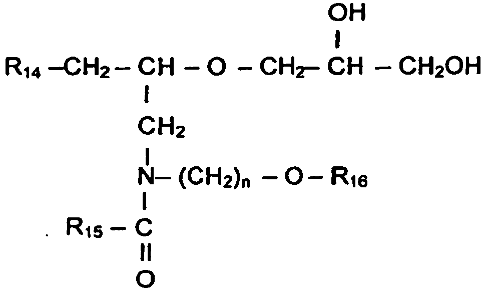 where R¹⁴ and R¹⁵ are independent from each other alkyl- or / alkenyl group with 10 to 22 carbon atoms, R¹⁶ is methyl, ethyl, n-propyl or isopropyl group and n is a number between 1 to 6, preferably 2 or 3.

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2 and especially 0.01 to 1% by weight calculated to the total weight of the composition. The fatty acids may be contained at a level of 0.01 to 2.5% and epecially 0.01 to 1% by weight calculated to the total weight of the composition. Phytosterol concentration of the conditioners is less than 1 % and preferably less than 0.5% by weight calculated to the total weight of the composition. It should be noted without limiting the use of those ingredients the effect of those hair restructuring ingredients is especially elevated when used in combination with penetration enhancers.

The compositions according to the invention can also contain dyestuffs for the direct dyeing of human hair. In this case, direct dyes are included into the compositions. As direct dyes, cationic and neutral nitro dyes are useful. The use of anionic dyes is as well possible though the color enhancing ability especially at higher end of pH specification is observed to be very low. The concentration of those dyes is in between 0.0001 - 5% by weight, preferably 0.0001 -2.5% by weight and more preferably 0.0001 - 1.5% by weight calculated to the total composition.

The useful cationic dyes according to the invention are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 and Basic Yellow 57. According to the invention, suitable cationic dyestuffs are in principal those any available on the market for cosmetic hair colouring applications. For this purpose, special reference is made to the PCT application WO 95/15144 of Ciba-Geigy AG.

Examples to suitable direct acting anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples to those suitable neutral dyes (HC dyes), so called nitro dyes, are HC Blue No.2, HC Blue No.4, HC Blue No.5, HC Blue No.6, HC Blue No.7, HC Blue No.8, HC Blue No.9, HC Blue No. 10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder.

The compositions are hair styling compositions used after shampooing and eventually conditioning hair. The compositions comprise in addition to the liquid Bamboo extract at least one film forming polymer.

The styling/film forming polymer or polymers is/are selected from the anionic, non-ionic, cationic and/or amphoteric or zwitterionic ones. The styling composition of the present invention comprises at least one styling polymer. Certainly, the similar effects are achieved when more than one polymer of the same type or as well of different types in combination are used.

Non-ionic polymers are selected from the ones soluble in water and/or alcohol and/or in alcohol water mixtures, at any ratio. Under the definition of soluble in alcohol and alcohol water mixture, it should be understood that the polymer is soluble in lower alcohols such as ethanol, n-propanol or isopropanol and in their mixtures with water, at any ratio

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64 from BASF AG.

Further non-ionic polymer suitable for compositions of the present invention is vinylpyrrolidone/vinylacetae/vinylpropionate copolymer known with the trade name Luviskol VAP 343 as well from BASF.

Natural non-ionic polymers are as well suitable for the composition of the present invention. Those are such as cellulose, chitosan, guar gum, neutralised shellac and their derivatives.

Cationic polymers are of greater variability in their structure found to be suitable for the styling composition of the present invention. The ones disclosed above as a conditioner are also suitable as hair styling polymer.

The cationic polymers also include the quatemized products of graft polymers from organopolysiloxanes and polyethyl oxazolines described in EP-A 524 612 and EP-A 640 643. Composition of the present invention preferably comprise an organopolysiloxane wherein at least one silicium atom is linked to an alkylene group having a hetero-atom, in particular a nitrogen atom, with poly-(N-acyl alkyleneimine) units of the formula 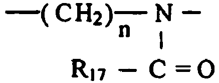 wherein n is a number from 1 to 5 and R₁₇ is hydrogen, a C₁-C₁₂-alkyl or cycloalkyl, aralkyl or aryl group.

Preferred organopolysiloxane polymers are those of the type disclosed in EP-A 640 643, in particular optionally quatemized aminoalkyl, in particular aminopropyl dimethyl polysiloxane/polyethyl oxazoline copolymers of the formula 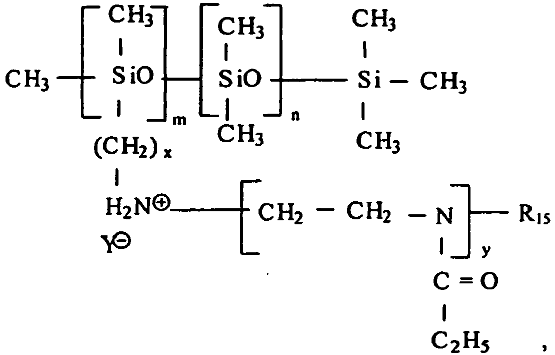 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl,
ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Amphoteric or zwitterionic polymers, preferably be used in mixture with at least one non-ionic and/or cationic polymers, are also found to be suitable for styling composition of the present invention. Examples to the preferred ones are copolimerisate of n-octylacrylamide, acrylic or methacrylic acid and tert.-butylaminoethylmethacrylate known with its trade name Amphomer, copolymer of methacryloylethylbetaine and alkyl methacrylate known as Yukaformer, terpolymer of methacrylic or acrylic acid and itaconoic acid and a basic monomer of mono or dialkylaminoalkyl acrylate or methacrylate or acryla of methacrylamide known with the trade name Aquaflex SF 40.

As amphoteric polymers which can be used alone or in mixture with at least one additional cationic and/or nonionic polymer, special reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Anionic polymers are as well suitable for styling compositions of the present invention. Suitable ones are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®";

Concentration of styling polymers of anionic, cationic, non-ionic and/or amphoteric or zwitterionic character is in the range of 0.5- 25%, preferably 0.5- 20% and most preferably 0.5 -15% by weight, calculated to the total composition.

The compositions according to the invention can comprise propellant and/or mixtures of propellants, preferably in an amount up to 50% by weight, calculated to the total aerosol foam composition, in the case that an aerosol preparation is produced. Useful propellants are, for example, hydrocarbons such as propane, n-butane, i-butane and the mixtures thereof, dimethyl ether, and/or carbon dioxide. The pressure in the aerosol container preferably ranges from about 4 to 6 bar.

The pH of the compositions according to the invention is in the range of 2 to 8, preferably 2 to 7, more preferably 3 to 7. For adjusting the pH of the said compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1.5% by weight calculated to the total composition. The pH of the composition can also be adjusted to the required pH by using alkaline solution such as sodium hydroxide, potassium hydroxide or their salts with those acids mentioned above in the case that at the selected acid concentration pH of the composition is lower than that of the aimed value.

The viscosity of the compositions depends on the type of application according to the invention.

For composition designed for styling purposes the viscosity values especially for gel type of preparations may be much higher than mentioned above. Waxes are practically solid and no viscosity values are given here.

In general the compositions of the present invention can be in the form of fully transparent, semitransparent or non-transparent. Transparency should certainly be judged by naked eye in a therefore suitable vessel.

Furthermore compositions of the present invention can comprise all substances customarily found in such preparations.

Examples of such substances are complexing agents, dyestuffs, preservatives, pH-regulants, viscosity regulants such as inorganic salts or polymers to the extent they are not already contained in the initial surfactant mixtures, fragrances, pearl-gloss agents, thickening agents, moisturizers. Furthermore, compositions dispensed from an aerosol preferably do not contain any preservative.

The following examples illustrate the invention.

The following Liquid extracts from Bambusa vulgaris were used in the examples:

| **Extract 1** | | **Extract 2** | |
|---|---|---|---|
| Isopropyl alcohol | 90% by weight | Propylene glycol | 70% by weight |
| Bambusa vulgaris | 5% | Bambusa vulgaris | 5% |
| PEG-8 | 5% | Sorbitol | 5% |
| | | Water | 20% |

In the examples below the liquid Bambusa vulgaris extracts are referred as Extract 1 or Extract 2.

### Example 1 (not in accordance with the invention)

### Spray conditioner - non-aerosol

| | |
|---|---|
| Ethanol | 45.0 (Gew.-%) |
| Polyquatemium-6 | 0.1 |
| Extract 2 | 0.2 |
| Ceramide compound according to formula (R¹⁴=C₁₅H₃₁; R¹⁵=C₁₆H₃₃; R¹⁶=H; n=2) | 0.1 |
| Behenic acid | 0.2 |
| Avocadin | 0.05 |
| PEG-160-hydriertes Ricinusöl | 0,5 |
| Parfum, Konservierungsmittel | q.s. |
| Citronensäure | ad pH 4.7 |
| Wasser | ad 100.0 |

The composition is applied to hair from a common pump spray. Hair treated with the composition has more elasticity, smoothness and shine. Leaving out Bambusa vulgaris extract resulted in loss of smoothness and shine. Upon repeated application hair structure improvements are as well observed. This is due to the content of restructuring ingredients.

### Example 2

### Foam conditioner

| | |
|---|---|
| Quatemium-80 | 0.2 (Gew.-%) |
| Polyquaternium-11 | 0.7 |
| Ceramide compound according to formula (R¹⁴=C₁₅H₃₁; R¹⁵=C₁₆H₃₃; R¹⁶=H; n=2) | 0.2 |
| Behenic acid | 1.0 |
| Extract 2 | 0.2 |
| Avocadin | 0.2 |
| PEG-160-hydriertes Ricinusöl | 0,5 |
| Parfum, Konservierungsmittel | q.s. |
| Citronensäure | ad pH 5 |
| Wasser | ad 100.0 |

The composition is filled in an aerosol can at a ratio of 90:10 with commonly used propellant, propan/butan mixture. The composition showed excellent hair shine effect. It should be noted that the composition could be used in two different ways, leave-in and rinse off. In leave-in application, amount used is obviously less than in the case of a rinse of application.

### Example 4

### Leave in conditioner for colored hair

| | |
|---|---|
| Dimethicone | 12,0 (Gew.-%) |
| Polyquaternium-37 | 2,0 |
| Extract 2 | 0.75 |
| Panthenol | 0.3 |
| Octinoxate/ ethylhexyl methoxycinnamate | 0,5 |
| Cetrimonium chloride | 0,3 |
| Caramel | 0,1 |
| Parfum, Konservierungsmittel | q.s. |
| Glyoxylic acid | ad pH 4,7 |
| Wasser | ad 100,0 |

Shine of coloured hair is especially improved with the above formulation. The formulation is especially suitable for leave-in application.

### Example 5

### Pumpspray

| | % |
|---|---|
| Ethanol | 90.0 |
| Polyquaternium-11 | 0.2 |
| Polyvinylcaprolactam | 1.0 |
| Extract 1 | 0.3 |
| Menthol | 0.3 |
| Fragrance | 0.2 |
| PEG-40 Hydrogenated Castor oil. | 0.2 |
| Water add | 100.0 |

Polymers are dissolved in ethanol, menthol, and fragrance is dissolved/mixed first with PEG-40 hydrogenated castor oil and than added to the solution of polymers in ethanol. Finally, the composition is made up to 100% by adding water.

### Example 6

### Aerosolspray

| | |
|---|---|
| Ethanol | 35.0 |
| PVP (Luviskol K 30) | 0.2 |
| Vinylacetate/crotonic acid copolymer | 3.6 (Aristoflex A 60) |
| Extract 2 | 2.0 |
| Fragrance | 0.2 |
| Water | 34.1 |
| Dimethylether | 38.0 |
| n-pentane | 12.0 |
| Neutralizing agent | q.s. pH 8.0 |

The composition is prepared in the same way as in Example 5.

### Example 7

### Pumpspray

| | |
|---|---|
| PVP (Luviskol K 90) | 0.3 |
| Acrylates/Octylacrylamide Copolymer | 1.0 |
| Aminomethyl Propanol | 0.25 |
| Fragrance | 0.2 |
| Extract 1 | 2.0 |
| Ethanol | add 100 |

The composition is prepared in the same way as in Example 5.

### Example 8

### Pumpspray

| | % |
|---|---|
| Polyquaternium-11 | 0.25 |
| VPNA Copolymer ((Luviskol VA 64W) | 2.0 |
| Extract 2 | 0.8 |
| Menthyl lactate | 0.2 |
| Ethanol | 40 |
| Fragrance | 0.2 |
| PEG-40 Hydrogenated Castor oil | 0.2 |
| Water | add 100 |

The composition is prepared in the same way as in Example 5.

### Example 8

### Pumpspray

| | % |
|---|---|
| Shellac (neutralized) | 1.0 |
| PVP | 0.2 |
| Menthol | 0.3 |
| Extract 1 | 0.8 |
| Octylmethoxycinnamate | 0.3 |
| Fragrance | 0.2 |
| Water | 10.0 |
| Ethanol | add 100 |

The composition is prepared in the same way as in Example 5.

### Example 10

### Pumpspray

| | % |
|---|---|
| Chitosan | 0.1 |
| VPNA Copolymer (Luviskol VA 64W) | 2.0 |
| Extract 1 | 0.5 |
| Fragrance | 0.2 |
| PEG-40 Hydrogenated castor oil | 0.2 |
| Ethanol | 10 |
| Water | add 100 % |

The composition is prepared in the same way as in Example 15.

### Example 11

### Styling gel

| | % |
|---|---|
| PVP (Luviskol K 90) | 4.0 |
| VP/VA Copolymer (Luviskol VA 64W) | 4.0 |
| Panthenol | 0.5 |
| Extract 2 | 1.5 |
| PEG-40 Hydrogenated castor oil | 0.2 |
| Glycerol | 10.0 |
| Ethanol | 10.0 |
| Fragrance, preservative | q.s. |
| Neutralizing agents | q.s to pH 6.0 |
| Water | add 100 |

### Example 12

### Styling gel

| | % |
|---|---|
| Acrylates/Octylacrylamide copolymer | 2.0 (Amphomer HC) |
| VPNA Copolymer (Luviskol VA 64W) Acrylates / C10-30 cetyl acrylate | 8.0 |
| Crospolymer | 0.6 |
| Panthenol | 0.5 |
| Extract 1 | 1.8 |
| PEG-40 Hydrogenated castor oil | 0.3 |
| Propylene glycol | 5.0 |
| Ethanol | 10.0 |
| Fragrance, preservative | q.s. |
| Neutralizing agents | q.s to pH 6.5 |
| Water | ad 100 |

### Example 13

### Styling mousse

| | % |
|---|---|
| Polyquaternium-11 | 2.0 (Gafquat 440) |
| VPNA Copolymer (Luviskol VA 64W) | 4.0 |
| Cetrimonium chloride | 0.4 |
| Laureth-23 | 0.2 |
| Extract 2 | 0.8 |
| PEG-40 Hydrogenated castor oil | 0.2 |
| Ethanol | 5.0 |
| Fragrance, preservative | q.s. |
| Water | ad 100 |

The composition is prepared in the same way as in Example 15. The solution is filled into an aerosol can with a propellant mixture of propane/butane at a liquid composition to propellant ratio of 90:10.

### Example 14

### Styling mousse

| | % |
|---|---|
| Polyquaternium-46 | 4.0 (Luviquat Hold) |
| VPNA Copolymer (Luviskol VA 64W) Dicocoylethyl hydroxyethylmonium | 8.0 |
| methosulfate | 0.6 |
| Laureth-23 | 0.3 |
| Extract 2 | 2.8 |
| PEG-40 Hydrogenated castor oil | 0.2 |
| Benzophenone-3 | 0.3 |
| Ethanol | 5.0 |
| Fragrance, preservative | q.s. |
| Water | ad 100 |

The solution is filled into an aerosol can with a propellant mixture of propane/butane at a liquid composition to propellant ratio of 95:5.

### Example 15

### Pump spray for damaged hair

| | |
|---|---|
| PVP (Luviskol K 90) | 0.3 |
| Acrylates/Octylacrylamide Copolymer | 1.0 |
| Aminomethyl Propanol | 0.25 |
| Ceramide compund according to formula (R¹⁴=C₁₅H₃₁; R¹⁵=C₆H₃₃; R¹⁶=H; n=2) | 0.05 |
| Behenic acid | 0.1 |
| Extract 1 | 0.8 |
| Avocadin | 0.05 |
| Fragrance | 0.2 |
| PEG-40 Hydrogenated castor oil | 0.2 |
| Ethanol | add 100 |

### Example 16

### Aerosol spray

| | |
|---|---|
| PVP (Luviskol K 90) | 2.5 |
| PEG/PPG-25/25 Dimethicne acrylates copolymer | 2.5 |
| Aminomethyl Propanol | q.s |
| Extract 1 | 1.0 |
| Polysilicone-9 | 0.5 |
| Fragrance | 0.2 |
| PEG-40 Hydrogenated castor oil | 0.2 |
| Ethanol | add 100 |

The above composition is filled at a weight ratio of 60 to 40 lotion to propellant mixture of Dimethylether and n-Pentane (1:1)

### Example 17

### Aerosol spray

| | |
|---|---|
| VPNA | 2.5 |
| Acrylates octylacrylamide copolymer | 5.0 |
| Aminomethyl Propanol | q.s |
| Extract 1 | 0.5 |
| Polysilicone-9 | 0.5 |
| Ethylmethoxycinnamate | 0.1 |
| Fragrance | 0.2 |
| PEG-40 Hydrogenated castor oil | 0.2 |
| Ethanol | add 100 |

The above composition is filled at a weight ratio of 45 to 55 lotion to propellant mixture of n-Pentane and Difluoro ethane (10:45)

### Example 18

### Aerosol spray

| | |
|---|---|
| Acrylates T-butylacrylamide copolymer | 14.0 |
| Aminomethyl Propanol | q.s |
| Extract 1 | 3.0 |
| Polysilicone-9 | 0.3 |
| Ethylmethoxycinnamate | 0.1 |
| Fragrance | 0.2 |
| PEG-40 Hydrogenated castor oil | 0.2 |
| Ethanol | add 100 |

The above composition is filled at a weight ratio of 55 to 45 lotion to propellant of n-Pentane.

### Example 19

### Styling Gel

| | |
|---|---|
| VPNA copolymer | 5.0 |
| Acrylates octylacrylamide copolymer | 1.0 |
| Acrylates/Palmeth-25 Acrylate Copolymer | 2.5 |
| Aminomethyl Propanol | q.s |
| Extract 1 | 3.0 |
| Polysilicone-9 | 0.3 |
| Benzophenone-3 | 0.1 |
| Panthenol | 0.3 |
| Fragrance | 0.2 |
| PEG-40 Hydrogenated castor oil | 0.2 |
| Ethanol | 10.0 |
| Water | add 100 |

### Example 20

### Styling Gel

| | |
|---|---|
| Acrylates octylacrylamide copolymer | 5.0 |
| Acrylates/Palmeth-25 Acrylate Copolymer | 2.0 |
| Acrylates copolymer | 2.0 |
| Aminomethyl Propanol | q.s |
| Extract 1 | 1.0 |
| Polysilicone-9 | 0.2 |
| Benzophenone-3 | 0.1 |
| Panthenol | 0.3 |
| Fragrance | 0.2 |
| PEG-40 Hydrogenated castor oil | 0.2 |
| Ethanol | 15 |
| Water | add 100 |

### Example 21

### Styling Gel

| | |
|---|---|
| Acrylates/Palmeth-25 Acrylate Copolymer | 1.0 |
| Acrylates copolymer | 3.0 |
| Aminomethyl Propanol | q.s |
| Extract 1 | 1.0 |
| Polysilicone-9 | 0.2 |
| Benzophenone-3 | 0.1 |
| Panthenol | 0.3 |
| Fragrance | 0.2 |
| PEG-40 Hydrogenated castor oil | 0.2 |
| Ethanol | 15 |
| Water | add 100 |

### Example 22

### Mousse

| | |
|---|---|
| Polyquaternium-16 | 1.5 |
| VPNA | 3.0 |
| Aminomethyl propanol | q.s |
| Extract 2 | 1.0 |
| Polysilicone-9 | 0.2 |
| Laureth-23 | 0.3 |
| Laureth-4 | 0.2 |
| Ethylhexylmethoxycinnamate | 0.1 |
| Panthenol | 0.3 |
| Fragrance | 0.2 |
| PEG-40 Hydrogenated castor oil | 0.2 |
| Ethanol | 5 |
| Water | add 100 |

The above formulation was filled with propane/butane as a propellant at a weight ratio of lotion to propellant of 90/10.

### Example 23

### Mousse

| | |
|---|---|
| Polyquaternium-11 | 0.3 |
| Polyqautemium-4 | 0.2 |
| VA Crotanes copolymer | 1.0 |
| Aminomethyl propanol | q.s |
| Extract 2 | 1.0 |
| Polysilicone-9 | 0.2 |
| Laureth-23 | 0.3 |
| Laureth-4 | 0.2 |
| Ethylhexylmethoxycinnamate | 0.1 |
| Panthenol | 0.5 |
| Fragrance | 0.2 |
| PEG-40 Hydrogenated castor oil | 0.2 |
| Ethanol | 12 |
| Water | add 100 |

The above formulation was filled with propane/butane as a propellant at a weight ratio of lotion to propellant of 92/8.

### Example 24

### Mousse

| | |
|---|---|
| Polyquatemium-46 | 0.6 |
| VP/VA | 8.0 |
| Aminomethyl propanol | q.s |
| Extract 2 | 4.0 |
| Polysilicone-9 | 0.5 |
| Laureth-23 | 0.1 |
| Laureth-4 | 0.2 |
| Ethylhexylmethoxycinnamate | 0.1 |
| Panthenol | 0.5 |
| Fragrance | 0.2 |
| PEG-40 Hydrogenated castor oil | 0.2 |
| Ethanol | 18 |
| Water | add 100 |

The above formulation was filled with propane/butane as a propellant at a weight ratio of lotion to propellant of 92/8.

### Example 25

### Hair Wax

| | % by weight |
|---|---|
| Mineral oil | 3.0 |
| Paraffin | 10.0 |
| Cetearyl alcohol | 2.0 |
| Ceteareth-20 | 4.0 |
| Glyceryl stearate | 3.0 |
| Acrylates/Palmeth-25 Acrylate Copolymer | 1.0 |
| Extract-2 | 5.0 |
| Polysilicone-9 | 1.0 |
| Fragrance, preservative, dye | q.s. |
| Water | to 100 |

### Example 26

| | Hair Wax |
|---|---|
| | % by weight |
| Ceteareth-3 | 20.0 |
| Candelila cera | 5.0 |
| Petrolatum | 40.0 |
| Glyceryl stearate | 3.0 |
| VP/VA | 6.0 |
| Extract-2 | 5.0 |
| Polysilicone-9 | 1.0 |
| Fragrance, preservative, dye | q.s. |
| Water | to 100 |

## Claims

1. Hair styling composition for hair **characterised in that** it comprises a liquid extract from Bambusa vulgaris, and at a concentration of 0.5 to 25% by weight, calculated to total composition.

2. Composition according to claim 1 **characterised in that** it comprises additionally at least one organopolysiloxane compound wherein at least one silicium atom is linked to an alkylene group having a hetero-atom, in particular a nitrogen atom, with poly-(N-acyl alkyleneimine) units of the formula 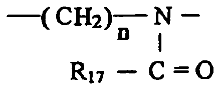 wherein n is a number from 1 to 5 and R₁₇ is hydrogen, a C₁-C₁₂-alkyl or cycloalkyl, aralkyl or aryl group.

3. Use of a composition according to claims 1 and 2 for enhancing hair shine.

## Patentansprüche

1. Haar Styling- Zusammensetzung für Haare, **dadurch gekennzeichnet, dass** sie einen flüssigen Extrakt von Bambusa Vulgaris und mindestens ein Film bildendes Polymer in einer Menge von 0,25 bis 25 Gew.- % enthält, berechnet auf die gesamte Zusammensetzung.

2. Zusammensetzung nach Anspruch 1, **dadurch gekennzeichnet, dass** sie zusätzlich mindestens eine Organopolysiloxanverbindung enthält, bei der mindestens ein Siliziumatom mit einer Alkylen- Gruppe, die ein Heteroatom, speziell ein Stickstoffatom hat, mit Poly-(N-Acylalkylenimin) Einheiten der Formel 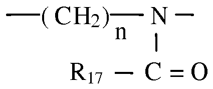 verbunden ist,
worin n eine Zahl von 1 bis 5 ist und R₁₇ Wasserstofff, eine C₁ - C₁₂-Alkyl- oder Cycloalkyl-, Aralkyl- oder Aryl- Gruppe ist.

3. Verwendung einer Zusammensetzung nach den Ansprüchen 1 und 2 zum Erhöhen der Leuchtkraft des Haares.

## Revendications

1. Composition pour le coiffage des cheveux, **caractérisée en ce qu'**elle comprend un extrait liquide de bambusa vulgaris et au moins un polymère filmogène d'une concentration de 0,5 à 25% en poids, calculée par rapport à la composition totale.

2. Composition selon la revendication 1, **caractérisée en ce qu'**elle comprend en plus au moins un composé organopolysiloxane dans lequel au moins un atome de silicium est relié à un groupe alkylène qui présente un hétéroatome, en particulier un atome d'azote, avec des unités poly-(N-acylalkylèneimine) de la formule 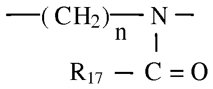 où n représente un nombre de 1 à 5 et R₁₇ représente l'hydrogène, un groupe C₁-C₁₂-alkyle ou cycloalkyle, aralkyle ou aryle.

3. Utilisation d'une composition selon les revendications 1 et 2 pour augmenter la brillance des cheveux.
